# FASCICULE DE BREVET EUROPEEN

(11) **EP 0 719 527 B1**
(45) Date de publication et mention de la délivrance du brevet: **29.08.2001**
(21) Numéro de dépôt: 95420357.6
(22) Date de dépôt: 11.12.1995
(51) Int. Cl.: A61F 2/00

(54) **Elément prothétique pour le traitement des hernies de l'aine, notamment par voie coelioscopique**
Protheseelement für die Behandlung von Leistenbrüchen, insbesondere für die Coelioskopie
Prosthetic element for the treatment of inguinal hernias, especially for celioscopic surgery

(30) Priorité: 30.12.1994 FR 9416019
(43) Date de publication de la demande: 03.07.1996
(73) Titulaire: Sgro, Jean-Claude, F-21000 Dijon (FR)
(72) Inventeur: Sgro, Jean-Claude, F-21000 Dijon (FR)
(74) Mandataire: Hubert, Philippe

(56) Documents cités:
- EP-A- 0 621 014
- US-A- 2 671 444
- US-A- 4 769 038

## Description

La présente invention concerne le traitement chirurgical des hernies, et plus particulièrement par coelioscopie.

Conformément au document US-A 469 038, pour le traitement chirurgical d'une hernie inguinale, avec un abord non précisé, on a déjà décrit un élément prothétique sous forme de plaque, comprenant un lien allongé et souple, destiné à se substituer au ligament inguinal, et trois feuillet solidarisés entre eux par ledit lien qui leur est commun, et s'étendant chacun à partir de ce dernier. Chacun de ces feuillets est réalisé en un matériau biocompatible, suffisamment lacunaire ou poreux pour supporter in vivo une implantation et croissance tissulaire, et suffisamment flexible pour être replié sur lui-même et déplié à volonté ; préférentiellement ce matériau consiste en un matériau textile. Une fois cet élément prothétique implanté en remplacement des structures musculo-aponévrotiques de la région inguinale, on peut distinguer ses différentes parties, selon :
- le lien fixé sur ou en remplacement du ligament inguinal, à une extrémité sur l'épine iliaque supérieure antérieure, et à l'autre extrémité sur le tubercule pubien ;
- un premier feuillet, supérieur et postérieur, comprenant une découpe comportant une lumière pour le passage du cordon spermatique ; de cette manière, ce feuillet enserre ledit cordon, après liaison l'une à l'autre des deux bords de la découpe, en dehors de la lumière précitée ; par ailleurs, ce feuillet est fixé sur ou en remplacement du fascia tranversalis ;
- une deuxième feuillet, supérieur et antérieur fixé sur ou en remplacement de l'aponévrose du muscle abdominal oblique externe ;
- et un troisième feuillet, inférieur, fixé sur ou en remplacement du ligament de Cooper, et éventuellement sur l'enveloppe de l'artère fémorale.

L'élément prothétique précédemment décrit et la technique chirurgicale qui lui est associée présentent différents inconvénients.

En pratique, pour éviter toute strangulation du cordon spermatique, on est conduit à laisser un espace interstitiel relativement important entre la lumière découpée dans le feuillet supérieur et postérieur, et ledit cordon. Cet espace interstitiel favorise en pratique une récidive de la hernie, par glissement d'un nouveau sac péritonéal le long du cordon spermatique. Cet inconvénient est aggravé lorsque la découpe refermée sur elle-même dans le feuillet supérieur postérieur se trouve distendue, ou lorsque l'orifice inguinal interne est très large, par exemple dans le cas de patients corpulents et obèses, et provoque une déformation en entonnoir du feuillet précité.

Le même feuillet supérieur et postérieur s'avère en pratique insuffisant pour résister à la pression des viscères, et oblitérer correctement l'orifice inguinal interne.

La technique chirurgicale précédemment décrite, de mise en oeuvre particulièrement longue, demeure exclusive au remplacement ou reconstruction du ligament inguinal interne, et des gaines musculaires et abdominales, à savoir du fascia tranversalis et de l'aponévrose du muscle grand oblique. L'indication de son utilisation demeure extrêmement rare, car elle suppose le remplacement pur et simple de structures anatomiques détruites par une chirurgie antérieure répétée. Et cette technique n'assure qu'une protection externe par rapport à l'orifice inguinal.

Conformément à la demande de brevet français 2 704 139, on a décrit et proposé un élément prothétique sous forme de plaque, notamment pour le traitement par coelioscopie de toute hernie, comprenant au moins deux feuillets réalisés en un matériau tel que défini précédemment liés l'un à l'autre de manière non jointive, par un renfort, obtenu notamment par tricotage, et créant une surépaisseur par rapport à la simple épaisseur de chacun desdits feuillets.

La présente invention a pour objet de remédier aux inconvénients de l'art antérieur, explicités par références au document US-C-4769 038.

En particulier, la présente invention a pour objet un élément prothétique du type de celui défini et décrit dans la demande de brevet français FR-A-2 704 139, pouvant constituer à la fois un renfort interne et un obstacle à toute récidive de la hernie, au niveau de l'orifice inguinal interne, et tel que défini à la revendication 1.

Un élément prothétique selon l'invention associe en coopération les caractéristiques techniques suivantes :
- le lien allongé et souple a la forme d'un renfort, dont la hauteur est adaptée pour obturer l'orifice inguinal interne
- un premier feuillet, se trouvant en position antérieure et inférieure une fois l'élément prothétique implanté, est celui destiné à enserrer le cordon spermatique, grâce à la découpe comportant la lumière pour le passage dudit cordon
- un deuxième feuillet, disposé en position supérieure une fois l'élément prothétique mis en place, s'étend de l'autre côté du renfort, avec une hauteur suffisante pour reposer sur la face interne du fascia transversalis, avec ou sans attache sur ce dernier
- et un troisième feuillet, se trouvant en position postérieure et inférieure une fois l'élément prothétique mis en place, à une hauteur suffisante pour être rabattu et maintenu contre la face interne du feuillet supérieur, puis rabattu contre le feuillet antérieur inférieur, en séparant le péritoine de l'espace résiduel dans le feuillet antérieur inférieur, entre le cordon spermatique et la lumière précitée.

Grâce à la présente invention, il est possible de réaliser à la fois un calibrage de la lumière assurant le passage du cordon spermatique et une pariétalisation de ce dernier.

Au surplus, un élément prothétique selon l'invention ne nécessite que la fermeture de la découpe, à l'exclusion de toutes autres attaches telles qu'agrafes, lesquelles peuvent néanmoins être utilisées dans cetaines indications.

La présente invention est maintenant décrite par référence au dessin annexé dans lequel :
- la figure 1 représente une vue en perspective d'un élément prothétique selon l'invention, dans sa conformation dépliée ou déployée, avec néanmoins le feuillet postérieur inférieur rabattu contre la face interne du feuillet supérieur ;
- la figure 2 représente une vue partielle en coupe verticale de l'élément prothétique représenté à la figure 1 ;
- les figures 3 à 7 représentent de face, et en vue partielle, différentes modalités d'exécution du feuillet antérieur inférieur d'un élément prothétique selon l'invention ;
- la figure 8 représente en perspective un élément prothétique selon l'invention, dans une conformation repliée, notamment adaptée pour le passage dans un trocart ;
- la figure 9 représente de manière schématique et en perspective, le mode d'intervention chirurgicale selon l'invention, avant insertion, déploiement et mise en place d'un élément prothétique selon l'invention ;
- la figure 10 représente de manière schématique l'élément prothétique selon l'invention, mis en place autour du cordon spermatique, avant de rabattre le feuillet postérieur inférieur contre le feuillet antérieur inférieur ;
- la figure 11 représente l'élément prothétique selon l'invention mis en place après avoir rabattu le feuillet postérieur inférieur contre le feuillet antérieur inférieur et le cordon spermatique ;
- la figure 12 représente une vue anatomique et éclatée de la région de l'aine d'un patient après implantation et mise en place d'un élément prothétique selon l'invention.

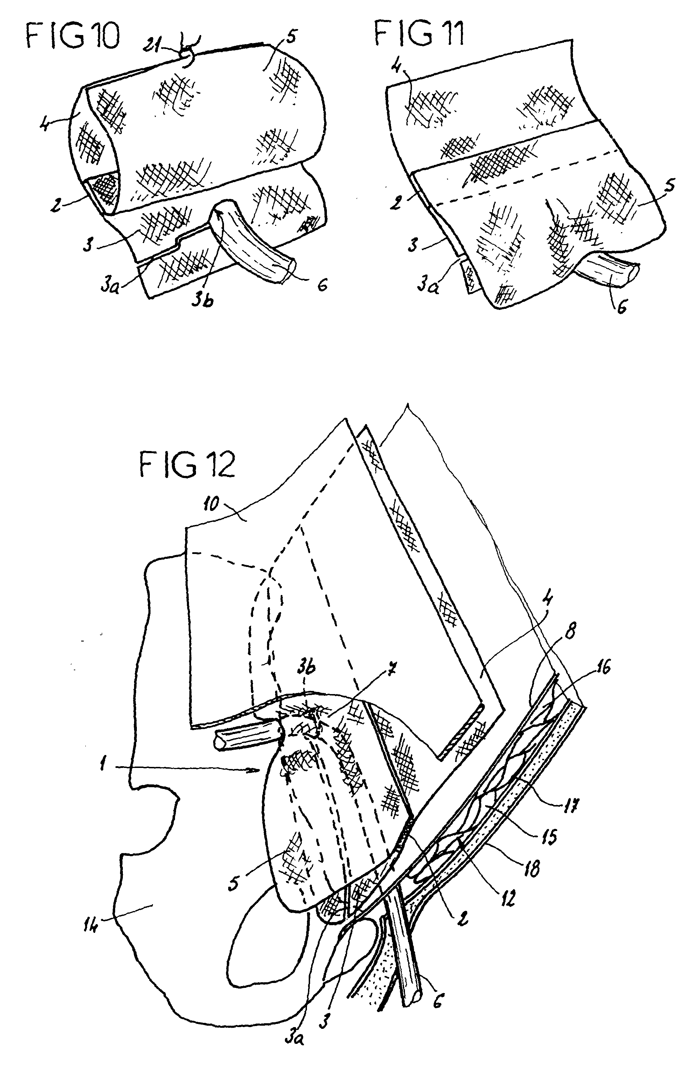

Conformément à l'invention, un élément prothétique 1 présente de manière générale la forme d'une plaque, et comprend :
- un lien 2 allongé et souple, ayant la forme d'un renfort, dont la hauteur est adaptée pour obturer l'orifice inguinal 7 (Cf figure 12) ;
- un premier feuillet 3, disposé de manière antérieure et inférieure une fois l'élément prothétique mis en place, en enserrant le cordon spermatique 6 (Cf figure 12) ; ce feuillet s'étendant à partir du renfort 2 a une hauteur suffisante pour circonscrire le cordon spermatique 6, et permettre ou comprendre une découpe 3a comportant une lumière 3b calibrée pour le passage du cordon 6 ;
- un deuxième feuillet 4, disposé de manière supérieure une fois l'élément prothétique mis en place, s'étendant de l'autre côté du renfort 2, avec une hauteur suffisante pour reposer sur la face interne du muscle transversal et du fascia transversalis 8 (Cf figure 12) ; ce deuxième feuillet s'étend à partir du renfort ;
- et un troisième feuillet 5, disposé de manière postérieure et inférieure une fois l'élément prothétique mis en place, avec une hauteur suffisante pour être successivement rabattu et maintenu contre la face interne du feuillet 4 supérieur, et rabattu contre le feuillet 3 antérieur inférieur, en séparant le péritoine 10 (Cf figure 12) de l'espace résiduel dans le feuillet 3 antérieur inférieur entre le cordon spermatique 6 et la lumière 3b ; ce feuillet s'étend lui aussi à partir du renfort 2.

Chacun des feuillets est réalisé en un matériau biocompatible, suffisamment lacunaire ou poreux pour supporter in vivo une implantation ou croissance tissulaire, et suffisamment flexible pour être replié sur lui-même et déplié ; préférentiellement il s'agit d'un matériau textile, par exemple obtenu par tissage et/ ou tricotage d'un fil polyester, ce matériau pouvant être finalement revêtu par une couche de collagène.

Les trois feuillets 3 à 5 sont liés entre eux par le renfort 2 qui leur est commun, et s'étendent chacun en hauteur à partir dudit renfort.

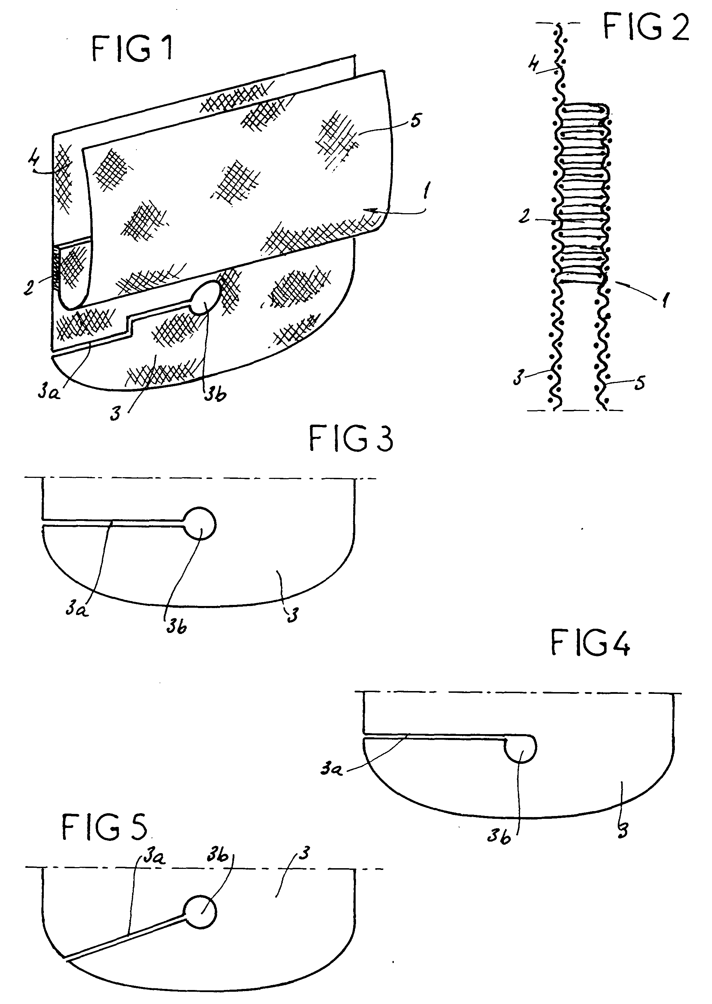

Conformément à la figure 2, le renfort 2 consiste en une liaison non jointive des trois feuillets 3 à 5 respectivement, obtenue notamment par tricotage, et créant ainsi une surépaisseur par rapport à la simple épaisseur de chacun des trois feuillets.

Comme montré par la figure 1 en particulier, l'élément antérieur inférieur 3 a une bordure 3c continûment arrondie, s'étendant d'une extrémité à l'autre du renfort 2.

Préférentiellement, mais de manière non exclusive, le dimensionnement de l'élément prothétique selon l'invention est le suivant :
- largeur de l'élément prothétique, c'est-à-dire dimension horizontale selon la figure 1, au moins égale à 10 cm, et préférentiellement au moins égale à 15 cm ;
- le renfort 2 a une épaisseur au moins égale à 0,1 cm, et préférentiellement égale à 0,2 cm, et une hauteur au moins égale à 1 cm, et préférentiellement au moins égale à 2 cm ;
- le feuillet antérieur inférieur a une hauteur (c'est-à-dire sa plus grande dimension), au plus égale à 10 cm, et préférentiellement au plus égale à 6 cm ;
- le feuillet supérieur 4 a une hauteur au moins égale à 4 cm, et préférentiellement au moins égale à 5,5 cm ;
- le feuillet postérieur inférieur a une hauteur au moins égale à 5 cm, et préférentiellement au moins égale à 7,5 cm.

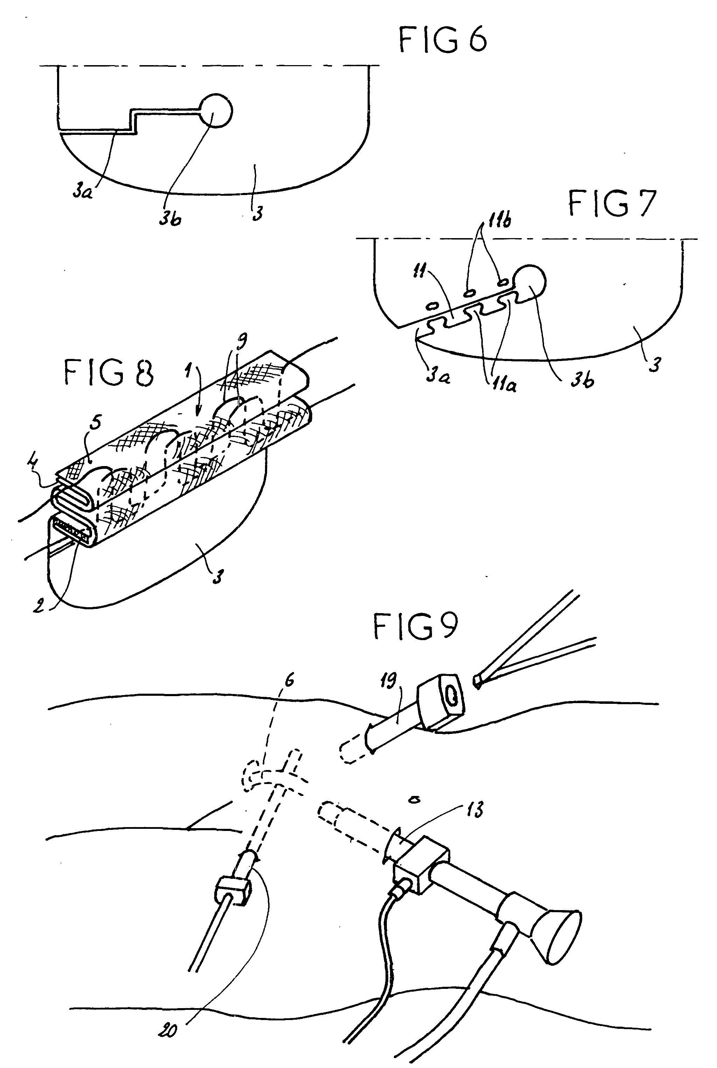

Préférentiellement, la découpe 3a dans le feuillet 3 antérieur inférieur a une forme choisie parmi les formes suivantes, à savoir :
- une forme parallèle au renfort 2 (Cf figure 3), éventuellement avec une lumière 3b décalée vers ou à l'opposé du renfort 2 (Cf figure 4) ;
- une forme oblique par rapport au renfort 2 (Cf figure 5) ;
- une forme en chicane à l'avant de la lumière 3b (Cf figure 6) ;
- et une forme en boutonnière 11, à l'avant de la lumière 3b, comportant des parties mâles lla sur l'un des bords de la découpe 3a, et des parties femelles 11b complémentaires sur l'autre bord de ladite découpe (Cf figure 7).

Conformément à la représentation de la figure 8, l'élément prothétique selon l'invention peut avoir une conformation repliée, selon laquelle au moins les feuillets 4 et 5 sont repliés ou roulés sur eux-mêmes, et sur le renfort 2, séparément ou ensemble, et sont maintenus dans cette conformation, par un fil amovible 9 entrelacé entre les différents plis de l'élément prothétique 1.

Avant de décrire la mise en place de l'élément précédemment défini, on se référera à la figure 12 montrant de manière schématique l'anatomie de l'aine, dans laquelle l'élément prothétique selon l'invention est implanté par la voie pré-péritonéale, pour traiter par coelioscopie une hernie inguinale oblique externe ; il s'agit de l'aine gauche.

Sur la figure 12 on reconnaîtra :
- l'os pelvien 14, sur lequel sont attachés le ligament inguinal et le ligament de Cooper, non représentés ;
- les muscles abdominaux oblique 15 et transversal 16, revêtus de manière externe par l'aponévrose 17 et de manière interne par le fascia transversalis 8, l'orifice inguinal interne 7, dans lequel passe le cordon spermatique 6 ;
- la peau abdominale 18 liée à une couche de graisse sous-cutanée ;
- le péritoine 10 qui contient les intestins non représentés.

Une hernie de l'aine résulte de la formation et du passage d'un sac péritonéal, sous l'effet de la pression des intestins, dans l'orifice ou canal inguinal interne 7.

De manière schématique, une telle hernie est traitée avec un élément prothétique selon l'invention, par chirurgie sous coelioscopie, comprenant les étapes suivantes :
a) comme représenté à la figure 9, grâce à un trocart 13, par pénétration d'un gaz stérile par la voie pré-péritonéale, on dissèque le sac pré-péritonéal entre le péritoine 10 et la paroi pariétale antérieure 12 ; deux trocarts 19 et 20 sont également placés sur la paroi pariétale intérieure, l'un 18 pour l'introduction de ciseaux par exemple, et l'autre 19 au-dessus et en dedans de l'épine iliaque antero-supérieure ;
b) on dispose de l'élément prothétique 1 tel que décrit précédemment, avec le feuillet postérieur inférieur 5 rabattu et maintenu contre la face interne du feuillet supérieur 4, par un lien 21 pouvant être résorbable (Cf figure 10) ; cet élément prothétique est en outre dans une conformation repliée ou roulée, telle que représentée à la figure 8, adaptée pour son passage dans le trocart 13 ;
c) on introduit l'élément prothétique 1 par le trocart 13, pour aboutir dans le sac pré-péritonéal, au voisinage de l'orifice inguinal interne 7 ; puis on déplie ou déploie l'élément prothétique dans le sac précité ;
d) comme représenté à la figure 10, on dispose le feuillet antérieur inférieur 3 de l'élément prothétique 1, de part et d'autre du cordon spermatique 6, puis on ferme la découpe 3a, de manière appropriée par exemple avec des agrafes ;
e) on fixe le feuillet supérieur 5 sur le fascia transversalis 8, en ayant positionné le renfort 2 de l'élément prothétique devant l'orifice inguinal interne 7 ;
f) en rompant le lien 21, et comme montré à la figure 11, on sépare le feuillet 5 inférieur et postérieur du feuillet supérieur 4, et on rabat ledit feuillet inférieur et postérieur dans une position antérieure séparant le cordon spermatique 6, et en particulier le passage interstitiel entre le cordon 6 et la lumière 3b, du péritoine 10 ;
g) on réduit le sac pré-péritonéal par exsufflation, de telle manière que le feuillet supérieur 5 serve de renfort, avec ou sans attache du type agrafe, du fascia transversalis, par appui du péritoine 10 contre l'élément prothétique 1.

Les mêmes étapes peuvent être réalisées par voie coelioscopique transpéritonéale, le décollement du péritoine se faisant depuis l'intérieur de la cavité abdominale. Le même élément prothétique peut être implanté par chirurgie traditionnelle, ouverte, pré-péritonéale.

Compte tenu de la forme et de la taille des feuillets antérieur inférieur et postérieur inférieur, l'indication d'un élément prothétique selon l'invention peut être élargie aux hernies inguinales directes, aux hernies crurales.

## Revendications

1. Elément (1) prothétique pour le traitement d'une hernie de l'aine, comprenant un lien (2) allongé et souple, ayant la forme d'un renfort, et trois feuillets (3, 4, 5) liés entre eux par ledit lien (2) qui leur est commun, et s'étendant chacun à partir dudit lien (2), lesdits feuillets étant réalisés en un matériau biocompatible, suffisamment lacunaire pour supporter in vivo une implantation tissulaire, et suffisamment flexible pour être replié sur lui-même et déplié, par exemple en un matériau textile,
- le lien (2) ayant une hauteur au moins égale à 1 cm, et préférentiellement au moins égale à 2 cm, adaptée pour obturer l'orifice inguinal (7),
- le premier feuillet (3) dit antérieur inférieur étant destiné à enserrer le cordon spermatique (6), avec une hauteur suffisante pour circonscrire ce dernier, et comprendre une découpe (3a) comportant une lumière (3b) pour le passage dudit cordon,
- le deuxième feuillet (4) dit supérieur s'étendant de l'autre côté du lien (2) avec une hauteur suffisante pour reposer sur la face interne du fascia transversalis (8),
- et le troisième feuillet (5) dit feuillet postérieur inférieur ayant une hauteur suffisante pour être rabattu et maintenu contre la face interne du feuillet (4) supérieur, puis rabattu contre le feuillet (3) antérieur inférieur, en séparant le péritoine (10) de l'espace résiduel dans le feuillet antérieur inférieur (3) entre le cordon spermatique (6) et la lumière (3b).

2. Elément prothétique selon la revendication 1, **caractérisé en ce que** le feuillet antérieur inférieur (3) a une bordure continûment arrondie, s'étendant d'une extrémité à l'autre du lien (2).

3. Elément prothétique selon la revendication 1, **caractérisé en ce qu**'il a une largeur au moins égale à 10 cm, et préférentiellement au moins égale à 15 cm.

4. Elément prothétique selon la revendication 1, **caractérisé en ce que** le lien (2) a une épaisseur au moins égale à 0,1 cm, et préférentiellement égale à 0,2 cm.

5. Elément prothétique selon la revendication 1, **caractérisé en ce que** le feuillet (3) antérieur inférieur a une hauteur au plus égale à 10 cm, et préférentiellement au plus égale à 6 cm.

6. Elément prothétique selon la revendication 1, **caractérisé en ce que** le feuillet (4) supérieur a une hauteur au moins égale à 4 cm, et préférentiellement au moins égale à 5,5 cm.

7. Elément prothétique selon la revendication 1, **caractérisé en ce que** le feuillet (5) postérieur inférieur a une hauteur au moins égale à 5 cm, et préférentiellement au moins égale à 7,5 cm.

8. Elément prothétique selon la revendication 1, dans une conformation repliée, notamment adaptée pour un passage dans un trocart, **caractérisé en ce qu**'au moins deux feuillets (4, 5) sont repliés ou roulés eux-mêmes et sur le lien (2), séparément ou ensemble, et sont maintenus dans la conformation repliée dudit élément prothétique, par un fil amovible (9) entrelacé dans les différents plis dudit élément prothétique.

9. Elément prothétique selon la revendication 1, **caractérisé en ce que** la découpe (3a) dans le feuillet antérieur inférieur (3) a une forme choisie parmi les formes suivantes, à savoir premièrement, parallèle (Fig. 3) au lien (2) éventuellement avec la lumière (3b) décalée (Fig. 4) vers ou à l'opposé du renfort, deuxièmement, oblique (Fig. 5) par rapport au lien (2), troisièmement en chicane (Fig. 6) à l'avant de la lumière (3b), et quatrièmement avec des boutonnières (11) (Fig. 7) à l'avant de la lumière, comportant des parties mâles (11a) sur l'un des bords de ladite lumière, et des parties femelles (11b) complémentaires sur l'autre bord de ladite lumière.

10. Elément prothétique selon la revendication 1, **caractérisé en ce que** le lien (2) consiste en une liaison non jointive des trois feuillets (3, 4, 5) respectivement, notamment par tricotage, créant une surépaisseur par rapport à la simple épaisseur de chacun des trois feuillets.

## Patentansprüche

1. Protheseelement (1) für die Behandlung eines Leistenbruchs, welches ein langgestrecktes und elastisches Bindeglied (2) in Form eines Verstärkungsteils sowie drei Lappen (3, 4, 5) aufweist, die mittels des ihnen gemeinsamen Bindegliedes (2) untereinander verbunden sind und von denen sich jedes von dem Bindeglied (2) aus erstreckt, wobei die Lappen aus einem Material, beispielsweise einem textilen Stoff, bestehen, der biologisch verträglich ist, in ausreichendem Maß Lücken zur Unterstützung bei einer Gewebeimplantation im lebenden Gewebe aufweist, und ausreichend flexibel ist, damit er umgeschlagen und auseinandergefaltet werden kann,
- wobei das Bindeglied (2) eine Höhe besitzt, die mindestens gleich 1 cm und vorzugsweise mindestens gleich 2 cm ist und sich zum Verschließen der Leistenöffnung (7) eignet,
- wobei der erste Lappen (3), der sogenannte anteriore untere Lappen, dazu bestimmt ist, den Samenstrang (6) mit einer ausreichenden Höhe zu umschließen, um letzteren zu umgrenzen, und einen Ausschnitt (3a) aufzuweisen, welche eine Öffnung (3b) für den Durchtritt des Strangs aufweist,
- wobei der zweite Lappen (4), der sogenannte obere Lappen, sich von der anderen Seite des Bindegliedes (2) aus mit einer Höhe erstreckt, die ausreicht, damit er auf der Innenfläche der *Fascia transversalis* (8) aufliegt,
- und wobei der dritte Lappen (5), der sogenannte posteriore untere Lappen, eine ausreichende Höhe aufweist, damit er umgeschlagen und gegen die Innenfläche der oberen Lamelle (4) gehalten und dann gegen den anterioren unteren Lappen (3) umgeschlagen werden kann, wobei er das Zwerchfell (10) vom übrigen Raum in dem anterioren unteren Lappen (3) zwischen dem Samenstrang (6) und der Öffnung (3b) trennt.

2. Protheseelement nach Anspruch 1, **dadurch gekennzeichnet**, dass der anteriore untere Lappen (3) einen durchgehend abgerundeten Rand aufweist, der sich von einem Ende des Bindegliedes (2) bis zum anderen Ende desselben erstreckt.

3. Protheseelement nach Anspruch 1, **dadurch gekennzeichnet**, dass es eine Breite aufweist, die mindestens gleich 10 cm und vorzugsweise mindestens gleich 15 cm ist.

4. Protheseelement nach Anspruch 1, **dadurch gekennzeichnet**, dass das Bindeglied (2) eine Breite von mindestens gleich 0,1 cm und vorzugsweise gleich 0,2 cm aufweist.

5. Protheseelement nach Anspruch 1, **dadurch gekennzeichnet**, dass der anteriore untere Lappen (3) eine Höhe aufweist, die höchstens gleich 10 cm und vorzugsweise höchstens gleich 6 cm ist.

6. Protheseelement nach Anspruch 1, **dadurch gekennzeichnet**, dass die obere Lamelle (4) eine Höhe aufweist, die mindestens gleich 4 cm und vorzugsweise mindestens gleich 5,5 cm ist.

7. Protheseelement nach Anspruch 1, **dadurch gekennzeichnet**, dass die posteriore untere Lappen (5) eine Höhe aufweist, die mindestens gleich 5 cm und vorzugsweise mindestens gleich 7,5 cm ist.

8. Protheseelement nach Anspruch 1 in umgeschlagener Ausformung, das insbesondere für den Durchtritt eines Trokars ausgebildet ist, **dadurch gekennzeichnet**, dass mindestens zwei Lappen (4, 5) voneinander getrennt oder zusammen um sich selbst umgeschlagen bzw. gerollt und auf das Bindeglied (2) aufgerollt sind, und in der umgeschlagenen Ausformung des Protheseelements von einem entfernbaren Draht bzw. Faden (9) gehalten sind, der in die verschiedenen Faltungen des Protheseelements eingearbeitet ist.

9. Protheseelement nach Anspruch 1, **dadurch gekennzeichnet**, dass der Ausschnitt (3a) in dem anterioren unteren Lappen (3) eine Form aufweist, die unter den folgenden Formen gewählt ist, nämlich zum ersten parallel (Fig. 3) zum Bindeglied (2), gegebenenfalls unter Versatz (Fig. 4) der Öffnung (3b) zur Verstärkung hin oder entgegengesetzt zu dieser, zum zweiten schräg (Fig. 5) bezüglich des Bindeglieds (2), zum dritten abgesetzt (Fig. 6) vor der Öffnung (3b), und zum vierten mit Knopflöchern (11) (Figl. 7) vor der Öffnung, wobei sie auf einer der Kanten der Öffnung vorstehende Abschnitte (11a) und auf der anderen Kante der Öffnung komplementäre Matrizenbereiche (llb) aufweist.

10. Protheseelement nach Anspruch 1, **dadurch gekennzeichnet**, dass das Bindeglied (2) aus einer nicht durchgängigen Verbindung von drei jeweiligen Lappen (3, 4, 5), insbesondere in Wirktechnik, besteht, wobei, bezogen auf die einfache Stärke jedes der drei Lappen, eine Überhöhung entsteht.

## Claims

1. A prosthetic element (1) for treating inguinal hernias, comprising an elongated and flexible link (2), in the form of a reinforcement, and three leaves (3, 4, 5) which are linked to each other *via* said link (2) which is common to them, and which extend, each one, from said link (2), said leaves being made from a biocompatible material, which is sufficiently lacunary in order to support a tissue implantation *in vivo,* and which is sufficiently flexible in order to be folded over itself and unfolded, for example made from a textile material,
- the link (2) having a height which is at least equal to 1 cm, and preferentially at least equal to 2 cm, and which is adapted in order to seal the inguinal opening (7),
- the first leaf (3) called the anterior inferior leaf being intended for tightly encircling the spermatic cord (6), with a height which is sufficient in order to surround the latter, and in order to comprise a cut-out (3a) comprising a lumen (3b) for the passing through of said cord,
- the second leaf (4) called the superior leaf extending from the other side of the link (2) with a height which is sufficient in order to lie on the internal face of the *fascia transversalis* (8),
- and the third leaf (5) called the posterior inferior leaf having a height which is sufficient in order to be folded up and maintained against the internal face of the superior leaf (4), and then folded down against the anterior inferior leaf (3), in separating the peritoneum (10) from the residual space in the anterior inferior leaf (3) between the spermatic cord (6) and the lumen (3b).

2. The prosthetic element according to claim 1, **characterised in that** the anterior inferior leaf (3) has a continuously rounded edge, extending from one extremity to the other of the link (2).

3. The prosthetic element according to claim 1, **characterised in that** it has a width at least equal to 10 cm, and preferentially at least equal to 15 cm.

4. The prosthetic element according to claim 1, **characterised in that** the link (2) has a thickness at least equal to 0.1 cm, and preferentially equal to 0.2 cm.

5. The prosthetic element according to claim 1, **characterised in that** the anterior inferior leaf (3) has a height at the most equal to 10 cm, and preferentially at the most equal to 6 cm.

6. The prosthetic element according to claim 1, **characterised in that** the superior leaf (4) has a height at least equal to 4 cm, and preferentially at least equal to 5.5 cm.

7. The prosthetic element according to claim 1, **characterised in that** the posterior inferior leaf (5) has a height at least equal to 5 cm, and preferentially at least equal to 7.5 cm.

8. The prosthetic element according to claim 1, in a folded conformation, notably which is adapted for passing into a trocar, **characterised in that** at least two leaves (4, 5) are folded or rolled up themselves and on the link (2), separately or together, and are maintained in the folded up conformation of said prosthetic element, by a detachable strand (9) intertwined in the various folds of said prosthetic element.

9. The prosthetic element according to claim 1, **characterised in that** the cut-out (3a) in the anterior inferior leaf (3) has a form selected from the following forms, namely firstly, parallel (Fig. 3) to the link (2) optionally with the lumen (3b) shifted (Fig. 4) towards or away from the reinforcement, secondly, oblique (Fig. 5) with respect to the link (2), thirdly in a zigzag (Fig. 6) in front of the lumen (3b), and fourthly with buttonholes (11) (Fig. 7) in front of the lumen, comprising male parts (11a) on one of the sides of said lumen, and complementary female parts (11b) on the other side of said lumen.

10. The prosthetic element according to claim 1, **characterised in that** the link (2) consists of non-joined bond of the three leaves (3, 4, 5) respectively, notably by knitting, creating an over-layer with respect to the single layer of each one of the three leaves.
